(19) Europäisches Patentamt
European Patent Office
Office européen des brevets

(11) **EP 0 465 708 B1**

(12) **EUROPÄISCHE PATENTSCHRIFT**

(45) Veröffentlichungstag und Bekanntmachung des Hinweises auf die Patenterteilung:
**22.05.1996 Patentblatt 1996/21**

(51) Int. Cl.$^6$: **A61B 5/00**

(21) Anmeldenummer: **90113287.8**

(22) Anmeldetag: **11.07.1990**

(54) **Anordnung zur elektrochemischen Bestimmung des Sauerstoffpartialdruckes pO2 in einem flüssigen Messmedium und Verfahren zum Betrieb der Anordnung**

Device for electrochemically determining the partial pressure of oxygen in a liquid and method for operating the device

Dispositif pour la détermination de la pression partielle d'oxygène dans un liquide et procédé d'opération du dispositif

(84) Benannte Vertragsstaaten:
**BE DE IT NL SE**

(43) Veröffentlichungstag der Anmeldung:
**15.01.1992 Patentblatt 1992/03**

(73) Patentinhaber: **SIEMENS AKTIENGESELLSCHAFT D-80333 München (DE)**

(72) Erfinder:
• **Gumbrecht, Walter, Dr.
  D-8552 Herzogenaurach (DE)**
• **Schelter, Wolfgang, Dr.
  D-8525 Uttenreuth (DE)**
• **Hofmann-Tikkanen, Roland
  D-8500 Nürnberg (DE)**
• **Preidel, Walter, Dr.
  D-8520 Erlangen (DE)**

(56) Entgegenhaltungen:
**EP-A- 0 273 258        EP-A- 0 353 328
US-A- 4 221 567        US-A- 4 535 786
US-A- 4 655 880**

EP 0 465 708 B1

**Beschreibung**

Die Erfindung bezieht sich auf ein Verfahren zum Betrieb einer Anordnung zur elektrochemischen Bestimmung des Sauerstoffpartialdruckes $pO_2$ in einem flüssigen Meßmedium, insbesondere Blut, mit einem Meßsensor, der zur Reduktion des Sauerstoffs $O_2$ mit einer Arbeitselektrode W (work) und einer Gegenelektrode C (counter) versehen ist. Zur Steuerung des Potentials der Elektroden ist eine Steuereinrichtung vorgesehen.

Mit Meßanordnungen zur elektrochemischen Bestimmung des Sauerstoffpartialdruckes $pO_2$ ist bekanntlich eine simultane Patientenüberwachung möglich. Die Kenntnis des Sauerstoffpartialdruckes ist beispielsweise eine notwendige Voraussetzung zur Beurteilung des Beatmungszustandes von künstlich beatmeten Patienten. Als Standardmethode zur Messung des Sauerstoffpartialdruckes $pO_2$ wird die polarographische Meßmethode (Reduktion von Sauerstoff) eingesetzt. Der Mechanismus dieser Sauerstoffreduktion wird wesentlich bestimmt von dem verwendeten Elektrodenmaterial sowie von der Vorbehandlung und der Zusammensetzung eines Meßelektrolyten. Als Arbeitselektrode W ist eine Kathode vorgesehen, die aus Platin bestehen kann, und als Anode, die als Gegenelektrode C dient, kann eine Ag/AgCl vorgesehen sein. Der Strom durch die Platinkathode beim Anlegen eines definierten Potentials) ist eine Funktion des Sauerstoffpartialdrucks $pO_2$ im Meßmedium. Ablagerungen aus dem Meßmedium an der Oberfläche der Platinkathode können jedoch zu einer Vergiftung dieser Elektrode führen; es ist deshalb ein ständiges Nacheichen des Elektrodensystems erforderlich.

Dieses Problem der Elektrodenvergiftung kann mit einer bekannten Ausführungsform teilweise gelöst werden, deren Elektrodensystem eine Platinkathode als Arbeitselektrode und eine Silber-Silberchloridanode Ag/AgCl enthält, die mit einem Elektrolyten versehen und mit einer hydrophoben Membran abgedeckt sind. Die Membran läßt einen Gasaustausch zwischen dem Meßmedium und dem Elektrolyten zu, verhindert aber das Eindringen von Substanzen, die zu einer Vergiftung der Elektroden führen können. Da bei der für die Messung notwendigen Reduktion von Sauerstoff in dem Elektrolyten zusätzliche Substanzen erforderlich sind oder dabei entstehen, muß für einen ausreichenden Vorrat dieses Elektrolyten gesorgt werden. Diese bekannte Ausführungsform ist somit für miniaturisierte Meßzellen, wie sie im Monitoring notwendig sind, nicht geeignet. Außerdem verhindert die verhältnismäßig große Zeitdauer, die verstreicht, bis sich zwischen dem Elektrolyten und einem Meßmedium ein Gleichgewicht einstellt, eine Messung verhältnismäßig schnell veränderlicher Sauerstoffpartialdrucke $pO_2$ (Clark in Trans. Am. Soc. Art. Int. Organs, 1956, 2, Seiten 41 bis 45).

In einer weiteren bekannten Ausführungsform zur elektrochemischen Bestimmung der Sauerstoffkonzentration ist ein Sauerstoffsensor mit unbedeckten Elektroden vorgesehen, die aus Edelmetall bestehen können. Die Arbeitselektrode kann beispielsweise aus Gold und die Gegenelektrode aus Platin bestehen. Um Vergiftungserscheinungen der Arbeitselektrode zu verhindern, werden dieser Elektrode zeitlich abwechselnd nacheinander zwei verschiedene Potentiale aufgeprägt. Die Meßphase ist klein gegenüber der Zyklusdauer. Der Zyklus besteht somit aus einer verhältnismäßig kurzen Meßphase und einer verhältnismäßig langen Regenerierphase. Als Meßsignal dient die an den Elektroden umgesetzte Ladung. Die Auswertung des Meßsignals beginnt mit einer zeitlichen Verzögerung von wenigstens einigen Millisekunden nach dem Beginn der Meßphase. Außer der Arbeitselektrode und der Gegenelektrode kann dieser Sauerstoffsensor auch noch mit einer Referenzelektrode versehen sein, die aus Silber/Silberchlorid Ag/AgCl bestehen kann. Die Oberfläche der Elektroden befindet sich sowohl während der Meßphase als auch während der Regenerierphase im Kontakt mit dem Meßmedium (EP-A2-0 170 998).

Der Erfindung liegt nun die Aufgabe zugrunde, diese bekannte Ausführungsform einer Anordnung zur Untersuchung eines flüssigen Meßmediums zu verbessern, insbesondere soll eine Gefahr der Vergiftung der Elektroden auch bei langer Betriebsdauer praktisch ausgeschlossen werden.

Eine bekannte Anordnung zur Untersuchung von Blut enthält eine Meßzelle mit einem Meßkanal, der mit einem Meßsensor und einem Referenzsensor versehen ist und der mit dem inneren Lumen eines Doppellumenkatheters verbunden ist. Das äußere Lumen des Katheders wird mit Infusionslösung versorgt. Beide Lumen haben eine gemeinsame Mündung, die in das Blut einführbar ist. Durch wiederholte Umkehrung der Strömungsrichtung im äußeren Lumen mittels einer umsteuerbaren Pumpe werden zeitlich nacheinander jeweils abwechselnd das Blut und die Infusionslösung über die beiden Sensoren gezogen, die im Meßkanal in einem vorbestimmten Abstand voneinander angeordnet sind. Von den beiden Sensoren befindet sich zeitlich nacheinander jeweils einer im Meßmedium und der andere in der Infusionslösung. Jeder der beiden Sensoren dient somit jeweils abwechselnd als Meßsensor und als Referenzsensor (US-Patent 4 841 974).

Die Erfindung macht Gebrauch von dieser bekannten Ausführungsform einer Anordnung zur Untersuchung eines flüssigen Meßmediums und sie besteht in einem Verfahren gemäß Anspruch 1. Während der verhältnismäßig kurzen Meßphase befinden sich die beiden Elektroden wenigstens im mittelbaren Kontakt mit dem Meßmedium, dagegen werden ihre Oberflächen während der verhältnismäßig langen Regenerierphase vom Spülmittel umspült, das zur elektrochemischen Bestimmung des Sauerstoffpartialdruckes $pO_2$ im Blut vorzugsweise eine Infusionslösung, insbesondere eine Ringerlösung, sein kann.

In einer besonderen Ausführungsform der Anordnung kann den beiden Elektroden des Meßsensors auch noch eine Referenzelektrode zugeordnet sein.

Ferner können die Elektroden auch noch durch eine hydrophile Membran abgedeckt sein.

Den Elektroden kann jeweils noch ein elektronischer Verstärker, vorzugsweise ein Operationsverstärker, zugeordnet sein, die an eine Steuereinrichtung angeschlossen sind. Als Steuereinrichtung kann vorzugsweise ein Computer, insbesondere ein Personalcomputer PC, vorgesehen sein.

Zur weiteren Erläuterung der Erfindung wird auf die Zeichnung Bezug genommen, in deren Figur 1 eine Anordnung zur Untersuchung eines flüssigen Meßmediums schematisch veranschaulicht ist. Figur 2 zeigt einen Querschnitt durch eine Meßzelle mit einem Meßsensor und in Figur 3 ist eine besondere Ausführungsform des Meßsensors schematisch veranschaulicht. In Figur 4 ist die Schaltung der Elektroden mit ihren zugeordneten elektronischen Verstärkern schematisch dargestellt. Die Figuren 5 bis 7 zeigen jeweils ein Diagramm zur Erläuterung der Wirkungsweise der Anordnung.

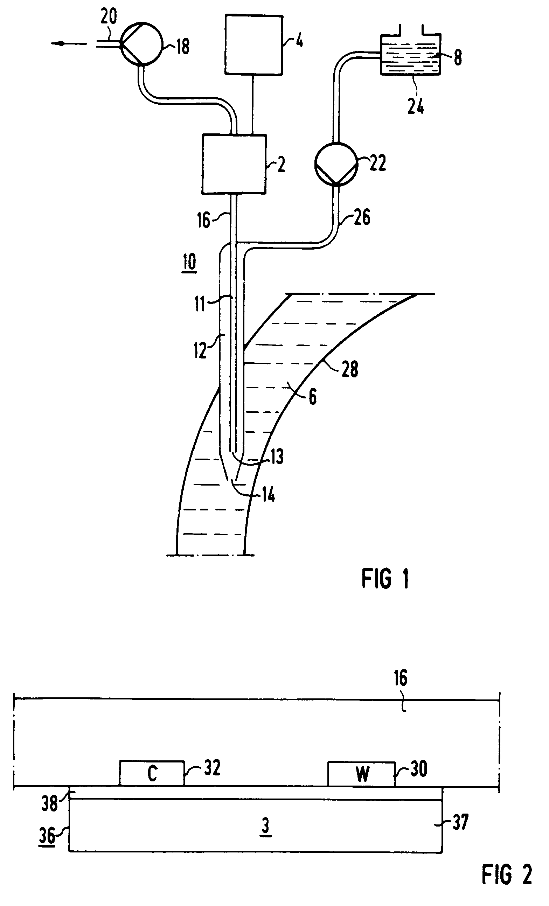

In einer Anordnung gemäß Figur 1 zur elektrochemischen Bestimmung des Sauerstoffpartialdruckes $pO_2$ in einem Meßmedium 6, insbesondere Blut, ist einer Meßzelle 2 eine elektronische Steuereinrichtung 4 zugeordnet, die vorzugsweise ein Computer, insbesondere ein Personalcomputer PC, sein kann. Die Meßzelle 2 gehört zu einem Probenahmesystem, das abwechselnd das Meßmedium 6 und ein Spülmedium 8 durch einen Meßkanal 16 strömen läßt. In diesem Probenahmesystem verbindet der Meßkanal 16 eine Vorrichtung 18 zur Steuerung der Strömungsgeschwindigkeit im Meßkanal 16 mit einem inneren Lumen 11 eines Mehrlumenkatheters mit wenigstens zwei Lumen, der zur Vereinfachung als Doppellumenkatheter 10 bezeichnet werden soll. Die Öffnung des inneren Lumens 11 ist in der Figur mit 13 bezeichnet. Das zweite Lumen 12 ist über eine umsteuerbare Pumpe 22 mit einem Behälter 24 für das Spülmedium 8 verbunden. Die beiden Lumen 11 und 12 haben eine gemeinsame Mündung 14. Die Vorrichtung 18 kann vorzugsweise aus einer Schlauchpumpe bestehen, die eine konstante Strömung im Meßkanal 16 gewährleistet. Ferner ist beispielsweise auch ein Drosselventil geeignet. Die Meßzelle 2, deren gesamte Ausdehnung im allgemeinen 10 mm nicht wesentlich überschreitet und insbesondere wesentlich weniger als 10 mm betragen kann und die vorzugsweise mit dem Doppellumenkatheter 10 eine gemeinsame Baueinheit bildet, stellt eine sehr kompakte Bauform der Anordnung dar, die im allgemeinen von der Vorrichtung 18 und der Steuereinrichtung 4 räumlich getrennt ist.

Die Meßzelle 2 enthält gemäß Figur 2 einen Meßsensor 3, dessen Arbeitselektrode W (working electrode) mit 30 und dessen Gegenelektrode C (counter electrode) mit 32 bezeichnet ist. Die Arbeitselektrode W und die Gegenelektrode C sind auf einem Substrat 3 angeordnet, das beispielsweise aus einem Siliziumhalbleiterkörper 37 bestehen kann, der an seiner Oberfläche mit einer Isolierschicht 38 versehen ist, die beispielsweise aus Siliziumdioxid $SiO_2$ bestehen kann. Die Arbeitselektrode W und die Gegenelektrode C sind in Dünnschichttechnik hergestellt und derart angeordnet, daß ihre freie Oberfläche im Meßkanal 16 wenigstens mittelbar im Kontakt ist mit dem Meßmedium 6 oder dem Spülmedium 8. Die Arbeitselektrode W, die im allgemeinen als Kathode geschaltet ist, kann vorzugsweise aus einem Edelmetall, insbesondere Platin, oder in einer besonderen Ausführungsform auch aus Kohlenstoff bestehen. Die Gegenelektrode C, welche in dieser besonders einfachen Ausführungsform zugleich die Funktion einer Referenzelektrode übernimmt, besteht aus Silber/Silberchlorid Ag/AgCl und ist im allgemeinen als Anode geschaltet. Die Dicke der beiden Elektroden, die vorzugsweise in Dünnschicht hergestellt sind, wird im allgemeinen 200 nm nicht wesentlich überschreiten.

Zur Inbetriebnahme der Anordnung zur elektrochemischen Bestimmung des Sauerstoffpartialdruckes im Meßmedium 6 gemäß den Figuren 1 und 2 wird zunächst das Spülmedium 8, vorzugsweise eine Infusionslösung, insbesondere eine Ringerlösung, mit Hilfe der Pumpe 22 durch einen Infusionsschlauch 26 in das äußere Lumen 12 des Doppellumenkatheters 10 bis in das Meßmedium 6, nämlich die Blutströmung einer Arterie 28 eines Patienten, gepumpt. Zugleich wird durch die Vorrichtung 18 über die Öffnung 13 des inneren Lumens 11 ein Teil dieses Spülmediums 8 durch den Meßkanal 16 des Meßsensors 2 gezogen und strömt somit an den Elektroden W und C des Meßsensors 3 entlang. Mit der Umkehrung der Strömung an der gemeinsamen Mündung 14, beispielsweise durch Umsteuerung der Pumpe 22, wird an der Mündung 13 des inneren Lumens 11 Blut aus der Arterie 28 gezogen, das in das innere Lumen 11 einströmt. Sobald eine vorbestimmte Menge des Meßmediums 6 in den Meßkanal 16 eingeströmt ist, wird die Pumpe 22 wieder umgepolt. Im Rhythmus der Umsteuerung der Pumpe 22 wird von der Vorrichtung 18 eine Folge von abwechselnd Meßmedium 6 und Spülmedium 8 durch den Meßkanal 16 der Meßzelle 2 gezogen, deren Gesamtmenge für einen Meßvorgang im allgemeinen etwa 20 µl nicht wesentlich überschreitet.

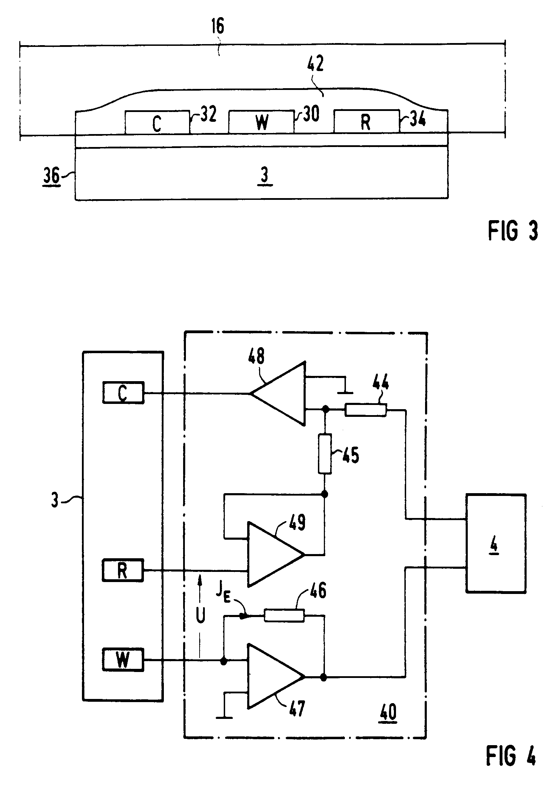

In einer weiteren Ausführungsform eines Meßsensors 3 gemäß Figur 3 kann das Substrat 36 neben der Arbeitselektrode W und der Gegenelektrode C noch mit einer Referenzelektrode R versehen sein, die in der Figur mit 34 bezeichnet ist und die im allgemeinen aus Silber/Silberchlorid Ag/AgCl besteht. In einer Ausführungsform kann die Gegenelektrode C aus Edelmetall, vorzugsweise Platin, bestehen. Diese Elektroden W, C und R können in einer bevorzugten Ausführungsform des Meßsensors 3 noch mit einer hydrophilen Membran 42 versehen sein, die als Schutz vor Verunreinigungen und Ablagerungen dient. Diese hydrophile Membran 42 kann beispielsweise aus Polyhydroxyethylmethacrylat (pHEMA) bestehen oder aus Poly(perfluoralkylen)-sulfonsäure (Nafion) bestehen. Das Meßmedium 6 und das Spülmedium 8 strömen jeweils abwechselnd im Meßkanal 16 an dieser hydrophilen Membran entlang. Aus dem Meßmedium 6 diffundiert während der Meßphase der Sauerstoff hindurch und wird an der Arbeitselektrode W umgesetzt

$$O_2 + 4\,e^{(-)} + 2H_2O \rightarrow 4\,OH^{(-)}.$$

Diese $O_2$-Reduktion während der Meßphase bewirkt eine Änderung des Elektrodenstromes $I_E$, die vorzugsweise nach elektronischer Verstärkung der Steuereinrichtung 4 zugeführt wird, wie es in Figur 4 schematisch veranschaulicht ist. Gemäß dieser Darstellung sind die Arbeitselektrode W, die Gegenelektrode C und die Referenzelektrode R jeweils über einen Verstärker mit der Steuereinrichtung 4 verbunden. In der Figur sind zwei Vorwiderstände mit 44 und 45 und ein Rückkopplungswiderstand mit 46 und ein als Strom-Spannungswandler wirkender Verstärker mit 47, ein als Addierer wirkender Verstärker mit 48 und ein als Impedanzwandler wirkender Verstärker mit 49 bezeichnet. Diese Verstärkerschaltung bildet einen Potentiostaten 40.

Der Potentiostat 40 kann in einer besonderen Ausführungsform der Anordnung monolithisch in den Siliziumhalbleiterkörper 37 integriert sein.

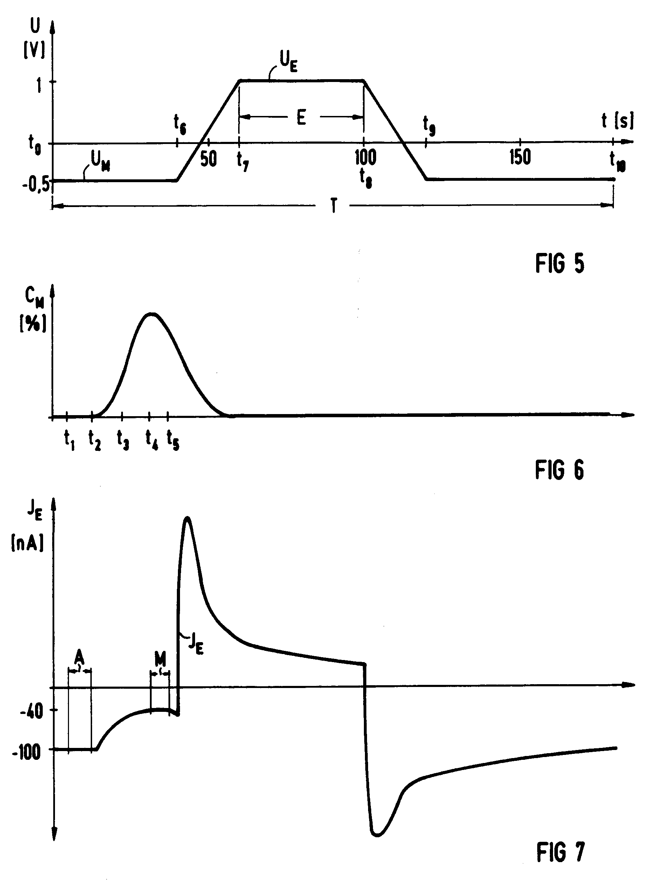

Gemäß dem Diagramm der Figur 5, in dem das Elektrodenpotential U in V über der Zeit t in Sekunden aufgetragen ist, beträgt das Elektrodenpotential der Arbeitselektrode W gegen die Referenzelektrode R (bei 0,1 mol $Cl^{(-)}$) beispielsweise $U_M = -0,5$ V. Gemäß Figur 6, in der die Konzentration $C_M$ des Meßmediums über der Arbeitselektrode 30 bzw. der Membran 42 in Abhängigkeit von der Zeit t aufgetragen ist, befinden sich die Elektroden im Spülmedium. Der Elektrodenstrom $I_E$ kann gemäß Figur 7, in dem der Elektrodenstrom $I_E$ in nA über der Zeit t aufgetragen ist, beispielsweise $I_E = -100$ nA betragen. Zur Zeit $t_0$ wird die Pumpe 22 umgesteuert auf Zug und zieht das Meßmedium 6 über die Mündung 14 und damit auch in das innere Lumen 11 des Katheters 10. Während der Zeit $t_1$ bis $t_2$, einer Eichphase A, befindet sich noch immer das Spülmedium am Sensor 3 und es beginnt die Eichung durch Messung des Elektrodenstromes. Am Ende der Eichphase A zur Zeit $t_2$ steigt gemäß Figur 6 die Konzentration $C_M$ des Meßmediums 6 am Sensor 3. Zur Zeit $t_3$ wird die Pumpe 22 umgesteuert auf Druck. Während einer verhältnismäßig kurzen Meßphase M von $t_4$ bis $t_5$ wird das relative Maximum des Elektrodenstromes $I_E$ ermittelt, das gemäß Figur 7 beispielsweise $I_E = -40$ nA betragen kann. Zur Zeit $t_6$ nach beispielsweise etwa 40 s erfolgt gemäß Figur 5 der Übergang vom Meßpotential beispielsweise $U_M = -0,5$ V zu einem Erholungspotential zur Zeit $t_7$ nach etwa 60 s von beispielsweise etwa $U_E = +1$ V. Von $t_7$ bis $t_8$ liegt während einer verhältnismäßig langen Regenerierphase E ein Erholungspotential von beispielsweise $U_E = 1$ V am Sensor 3. Während dieser Regenerierphase E ist gemäß Figur 6 die Konzentration am Sensor 3 Null und die Elektroden befinden sich im Spülmedium. In dieser Zeitspanne werden an der Arbeitselektrode adsorbierte Stoffe, die zur Elektrodenvergiftung führen können, desorbiert und somit die Elektrode regeneriert. Zur Zeit $t_8$ nach beispielsweise etwa 100 s erfolgt bis $t_9$ der Übergang vom Erholungspotential $U_E$ zum Meßpotential $U_M$. Nach einer Periode T von beispielsweise etwa 180 s beginnt zur Zeit $t_{10}$ ein neuer Meßzyklus.

Im Ausführungsbeispiel ist Blut als Meßmedium vorgesehen. Die Anordnung kann jedoch auch zur Bestimmung des Sauerstoffgehalts in anderen Meßmedien, beispielsweise Wasser, verwendet werden.

**Patentansprüche**

1. Verfahren zum Betrieb einer Anordnung zur elektrochemischen Bestimmung des Sauerstoffpartialdruckes ($pO_2$) in einem flüssigen Meßmedium, insbesondere Blut, mit einem Meßsensor, der zur Reduktion des Sauerstoffs ($O_2$) mit einer Arbeitselektrode (W) und einer Gegenelektrode (C) versehen ist, wobei

   - die Elektroden (30, 32) als Mikrostruktur in Dünnschichttechnik ausgeführt und auf einem Substrat angeordnet sind und wenigstens mittelbar einen Teil der Wand eines Meßkanals (16) bilden, dem abwechselnd während einer Meßphase das Meßmedium (6) und während einer Regenerierphase ein Spülmedium (8) zugeführt wird,
   - der Meßkanal (16) eine Vorrichtung (18) zur Steuerung der Strömungsgeschwindigkeit des Meßmediums (6) im Meßkanal (16) mit dem einen Lumen (11) eines Doppellumenkatheters (10) verbindet,
   - dem zweiten Lumen (12) des Doppellumenkatheters (10) das Spülmedium (8) mit umkehrbarer Strömungsrichtung zugeführt wird,
   - beide Lumen (11, 12) eine gemeinsame Mündung (14) haben, die in das Meßmedium (6) eingeführt wird, und
   - von den Elektroden (30, 32) während der Meßphase (MP) die Arbeitselektrode (30) als Kathode und die Gegenelektrode (32) als Anode geschaltet wird und während der Regenerierphase die Gegenelektrode (32) als Kathode und die Arbeitselektrode (30) als Anode.

2. Verfahren nach Anspruch 1, **dadurch gekennzeichnet,** daß den Elektroden (30, 32) eine Referenzelektrode (34) zugeordnet wird.

3. Verfahren nach Anspruch 1 oder 2, **dadurch gekennzeichnet,** daß die Elektroden (30, 32, 34) durch eine hydrophile Membran (42) abgedeckt werden.

4. Verfahren nach einem der Ansprüche 1 bis 3, **dadurch gekennzeichnet,** daß eine Arbeitselektrode (30) aus Platin eingesetzt wird.

5. Verfahren nach einem der Ansprüche 1 bis 3, **dadurch gekennzeichnet,** daß eine Arbeitselektrode (30) aus Kohlenstoff eingesetzt wird.

## Claims

1. Method for operating a device for electrochemically determining the partial pressure of oxygen ($pO_2$) in a liquid measuring medium, in particular blood, having a measuring sensor which is provided with a working electrode (W) and a counter electrode (C) for the reduction of the oxygen ($O_2$), wherein

   - the electrodes (30, 32) are designed as microstructure with thin-layer technology and are arranged on a substrate and form at least indirectly a part of the wall of a measuring channel (16), to which there are supplied alternately the measuring medium (6) during a measuring phase and a rinsing medium (8) during a regenerating phase,
   - the measuring channel (16) connects a device (18) for controlling the flow speed of the measuring medium (6) in the measuring channel (16) to the one lumen (11) of a double lumen catheter (10),
   - the rinsing medium (8) is supplied to the second lumen (12) of the double lumen catheter (10) with reversible flow direction,
   - both lumens (11, 12) have a common opening (14) which is inserted into the measuring medium (6), and
   - of the electrodes (30, 32) during the measuring phase (MP) the working electrode (30) is connected as cathode and the counter electrode (32) as anode and during the regenerating phase the counter electrode (32) as cathode and the working electrode (30) as anode.

2. Method according to claim 1, characterized in that a reference electrode (34) is associated with the electrodes (30, 32).

3. Method according to claim 1 or 2, characterized in that the electrodes (30, 32, 34) are covered by a hydrophilic membrane (42).

4. Method according to one of claims 1 to 3, characterized in that a working electrode (30) of platinum is used.

5. Method according to one of claims 1 to 3, characterized in that a working electrode (30) of carbon is used.

## Revendications

1. Procédé pour le fonctionnement d'un agencement pour la détermination électrochimique de la pression partielle d'oxygène ($pO_2$) dans un milieu de mesure liquide, en particulier le sang, à l'aide d'un capteur qui est muni, pour la réduction de l'oxygène ($O_2$), d'une électrode de travail (W) et d'une contre-électrode (C), dans lequel

   - les électrodes (30, 32) sont réalisées sous forme d'une microstructure réalisé selon la technique en couche mince et sont disposées sur un substrat et forment, au moins indirectement, une partie de la paroi d'un canal de mesure (16) auquel on achemine alternativement, pendant une phase de mesure, le milieu de mesure (6) et, pendant une phase de régénération, un milieu de rinçage (8),
   - le canal de mesure (16) relie un dispositif (18) pour la commande de la vitesse d'écoulement du milieu de mesure (6) dans le canal de mesure (16) au premier lumen (11) d'un cathéter (10) à double lumen,
   - on achemine le milieu de rinçage (8) au second lumen (12) du cathéter (10) à double lumen avec une direction d'écoulement apte à être inversée,
   - les deux lumens (11, 12) possèdent une embouchure commune (14) que l'on introduit dans le milieu de mesure (6), et
   - parmi les électrodes (30, 32), on met en circuit, pendant la phase de mesure (MP), l'électrode de travail (30) comme cathode et la contre-électrode (32) comme anode, et pendant la phase de régénération, la contre-électrode (32) comme cathode et l'électrode de travail (30) comme anode.

2. Procédé selon la revendication 1, caractérisé en ce qu'une électrode de référence (34) est attribuée aux électrodes (30, 32).

3. Procédé selon la revendication 1 ou 2, caractérisé en ce que les électrodes (30, 32, 34) sont recouvertes par une membrane hydrophile (42).

4. Procédé selon l'une quelconque des revendications 1 à 3, caractérisé en ce qu'on met en oeuvre une électrode de travail (30) en platine.

5. Procédé selon l'une quelconque des revendications 1 à 3, caractérisé en ce qu'on met en oeuvre une électrode de travail (30) en carbone.

FIG 1

FIG 2

FIG 3

FIG 4

FIG 5

FIG 6

FIG 7